# EUROPEAN PATENT APPLICATION

(11) **EP 3 581 926 A1**
(43) Date of publication of application: **18.12.2019**
(21) Application number: 19180363.4
(22) Date of filing: 14.06.2019
(51) Int. Cl.: G01N 27/414, B01L 3/00, G01N 33/49, G01N 33/94

(54) **INTEGRATED MICROFLUIDIC ORGANIC ELECTROCHEMICAL TRANSISTOR BIOSENSORS FOR DRUG LEVEL DETECTION**

(30) Priority: 14.06.2018 US 201862684895 P
(71) Applicant: The Charles Stark Draper Laboratory, Inc., Cambridge, Massachusetts 02139 (US)
(72) Inventor: Dow, Parker, Boston, MA 02118 (US); Sprachmann, Melissa, Somerville, MA 02143 (US); Zhang, Hongmei, Lexington, MA 02421 (US); Mutha, Heena, Somerville, MA 02143 (US); Kumar, Parshant, Stoneham, MA 02180 (US)
(74) Representative: FRKelly

(57) **Abstract**

The present disclosure describes a microfluidic system to rapidly detect a level of a drug present in a fluid sample. The system can be used to monitor drug levels in the blood of a patient to whom the drug has been prescribed. The microfluidic system can include one or more organic electrochemical transistors that are functionalized with a coating that may include aptamers or antibodies. The coating can bind or otherwise interact with the drug of interest to change the transconductance of the organic electrochemical transistors. The system can detect a change in the transconductance of the organic electrochemical transistors and signal the presence of the drug.

## Description

### CROSS-REFERENCE TO RELATED APPLICATION

This application claims priority to United States Provisional Patent Application No. 62/684,895, filed on June 14, 2018 and entitled "INTEGRATED MICROFLUIDIC ORGANIC ELECTROCHEMICAL TRANSISTOR BIOSENSORS FOR DRUG LEVEL DETECTION," which is hereby incorporated by reference in its entirety.

### BACKGROUND OF THE DISCLOSURE

It can be useful to monitor drug levels in the blood of a patient. For example, medical conditions such as epilepsy can be managed through administration of drugs, which may only be safe or effective when they are present at predetermined levels in the blood of a patient. Drug level detection and testing can be time consuming and labor intensive.

### SUMMARY OF THE DISCLOSURE

At least one aspect of this disclosure is directed to a microfluidic device for detecting an analyte in a fluid sample. The microfluidic device can include a substrate defining a flow channel configured to transport the fluid sample from an inlet of the flow channel to an outlet of the flow channel. The microfluidic device can include an organic electrochemical transistor (OECT) including a source, a drain, a transistor channel, and a gate. At least one of the transistor channel or the gate of the OECT can overlap a portion of the flow channel to contact the fluid sample in the flow channel. The microfluidic device can include a coating applied to at least one of the transistor channel or the gate. The coating can include an aptamer or an antibody selected to bind with the analyte to change a conductivity of the transistor channel or a work function of the gate. The microfluidic device can include a sensor configured to receive an electrical output of the OECT and to detect a level of the analyte within the fluid sample based on the electrical output of the OECT.

In some implementations, the microfluidic device can include a first separation region positioned in the flow channel between the inlet and the OECT. The first separation region can be configured to remove cells from the fluid sample before the fluid sample flows to the OECT. In some implementations, the first separation region can include a separation outlet configured to receive a portion of the fluid sample containing the cells and to transport the portion of the fluid sample containing the cells away from the flow channel. In some implementations, the first separation region can further include an acoustic wave generator configured to impart a standing wave across the first separation region to direct the portion of the fluid sample containing the cells toward the separation outlet. In some implementations, the microfluidic device can further include a second separation region positioned in the flow channel between the first separation region and the OECT. The second separation region can be configured to remove bacteria from the fluid sample before the fluid sample flows to the OECT.

In some implementations, the transistor channel of the OECT can include a conductive polymer material. In some implementations, the transistor channel of the OECT can include poly(3,4-ethylenedioxythiophene) polystyrene sulfonate (PEDOT:PSS).

In some implementations, the gate of the OECT can further include a gate electrode including a gold surface positioned within the flow channel of the microfluidic device. In some implementations, a majority of a length of the flow channel can have a first width. A region of the flow channel surrounding the gate of the OECT can have a second width that is at least twice the first width. In some implementations, the transistor channel of the OECT can have a length between 200 microns and 350 microns and a width between 2 millimeters and 6 millimeters. In some implementations, the gate of the OECT can have a rectangular shape with a length between 1 millimeter and 10 millimeters and a width between 3 millimeters and 7 millimeters.

In some implementations, the substrate can include at least one of glass, polydimethylsiloxane (PDMS), and acrylic. In some implementations, the analyte can include a small molecule drug. In some implementations, the small molecule drug can be carbamazepine.

Another aspect of this disclosure is directed to a method of fabricating a device for detecting an analyte in a fluid sample. The method can include forming a first sacrificial layer on a surface of a substrate. The first sacrificial layer can be patterned for the deposition of a source electrode and a drain electrode of a transistor. The method can include depositing a layer of conductive material over the first sacrificial layer. The method can include patterning the layer of conductive material to define the source electrode and the drain electrode of the transistor. The method can include forming a second sacrificial layer over the substrate. The second sacrificial layer can be patterned for the deposition of a transistor channel. The method can include depositing a conductive polymer material over the second sacrificial layer. The method can include patterning the conductive polymer material to define the transistor channel. The method can include functionalizing a gate electrode of the transistor with a coating including an aptamer or an antibody selected to bind with the analyte to change a work function of the gate electrode. The method can include positioning the gate electrode within a microfluidic channel containing the fluid sample with the analyte.

In some implementations, the method can include introducing the fluid sample containing the analyte into an inlet of the microfluidic channel. In some implementations, the method can include receiving an electrical output of the transistor. In some implementations, the method can include detecting or calculating a level of the analyte within the fluid sample based on the electrical output of the transistor.

In some implementations, depositing the layer of conductive material over the first sacrificial layer can include depositing a layer of gold. In some implementations, depositing the conductive polymer material over the second sacrificial layer can include depositing a layer of PEDOT:PSS.

In some implementations, the method can include cleaning a surface of the gate electrode using at least one of oxygen plasma cleaning or electrochemical cleaning. In some implementations, the method can include applying the coating to the surface of the gate electrode after the surface of the gate electrode is cleaned. In some implementations, the method can include removing the first sacrificial layer and removing the second sacrificial layer.

### BRIEF DESCRIPTION OF THE DRAWINGS

The accompanying drawings are not intended to be drawn to scale. Like reference numbers and designations in the various drawings indicate like elements. For purposes of clarity, not every component may be labeled in every drawing. In the drawings:
FIG. 1 illustrates a block diagram of an example system to detect drug levels in a fluid sample.
FIG. 2 illustrates a top view of an embodiment of the organic electrochemical transistor sensor that can be used in the system illustrated in FIG. 1.
FIG. 3 illustrates a block diagram of an example system to detect drug levels in a fluid sample.
FIG. 4 illustrates a schematic view of an embodiment of the organic electrochemical transistor sensor that can be used in the systems illustrated in FIGS. 1 and 2.
FIG. 5A illustrates a top view of an embodiment of the organic electrochemical transistor sensor that can be used in the system illustrated in FIG. 1.
FIG. 5B illustrates a perspective view of two of the organic electrochemical transistor sensors of FIG. 5A arranged in parallel.
FIG. 5C illustrates an exploded view of an electrode fixture that can be used in connection with the electrochemical transistor sensor of FIG. 5A.
FIG. 6 illustrates a flowchart of an embodiment a method for fabricating a device for detecting an analyte in a fluid sample.
FIGS. 7A-7F illustrate stages of construction of a portion of an example device that can be fabricated according to the method of FIG. 6.
FIG. 8 illustrates a cross-sectional view of a gate electrode of an example OECT sensor functionalized with an aptamer, which can be used in the systems illustrated in FIGS. 1 and 2.
FIGS. 9A-9C illustrates cross-sectional views of stages of functionalization of a gate electrode of an example OECT sensor functionalized with an antibody, which can be used in the systems illustrated in FIGS. 1 and 2.

### DETAILED DESCRIPTION

The various concepts introduced above and discussed in greater detail below may be implemented in any of numerous ways, as the described concepts are not limited to any particular manner of implementation. Examples of specific implementations and applications are provided primarily for illustrative purposes.

The present disclosure describes systems capable of rapidly detecting a presence or a level of an analyte within a fluid sample. For example, the systems and methods of this disclosure can be capable of determining drug levels in fluid sample, while maintaining a low limit of detection. The systems can include organic electrochemical transistors (OECTs). The OECTs can include a channel material or gate electrode coated with a coating selected for detection of a given analyte. The coating can serve as a drug detection element. Thus, the analyte can be a drug, and the coating can be configured to bind with the drug of interest, such as an anti-epilepsy drug (AED). The coating can include an aptamer or an antibody. Binding of the analyte to the coating can induce a gate voltage change, which can result in a change in the source and drain current of the OECT. The magnitude in the current change can indicate the amount of analyte present in a test sample. The OECTs can be incorporated into microfluidic devices to provide rapid detection of such analytes, which may include small molecule drugs.

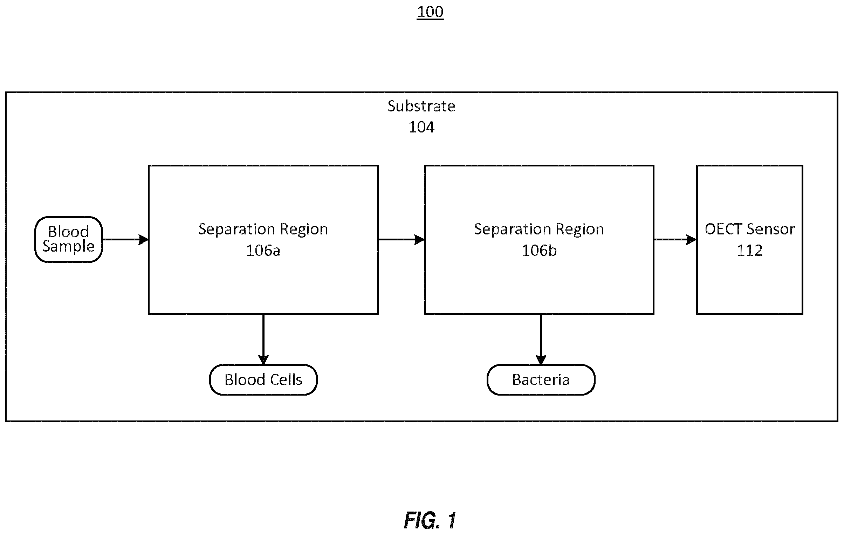

FIG. 1 illustrates a top view of an example system 100 to detect drug levels in a fluid sample. The system 100 can be, for example, a microfluidic device fabricated on a substrate 104. The system 100 can include a flow channel configured to transport a blood sample from left to right in the depiction of FIG. 1. The system 100 can include multiple separation regions 106. Each of the separation regions 106 can receive fluid from an upstream portion of the system 100, and can be configured to filter out or otherwise remove a portion of the sample. A microfluidic flow channel receive a fluid sample (e.g., a blood sample) and can transport the fluid sample along a length of the flow channel, terminating at an OECT sensor 112. The OECT sensor 112 can be defined, for example, within a well of the substrate 104. The OECT sensor 112 can be configured to detect a presence or a level of an analyte such as a drug within a fluid sample, such as a blood sample. The drug can be a small molecule drug such as a drug for treating patients diagnosed with epilepsy. For example, the drug can be carbamazepine (CBZ), Ampicillin, or Tenofovir.

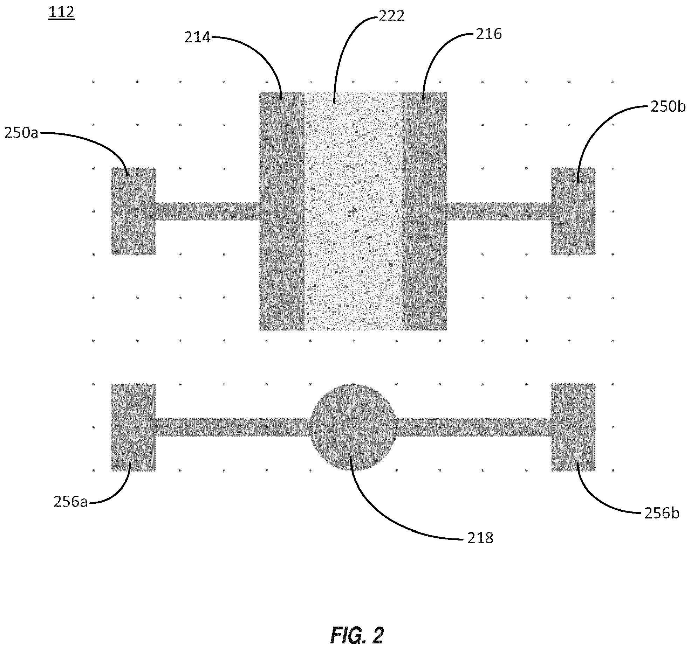

Referring now to FIG. 2, a top view of an embodiment of the OECT sensor 112 that can be used in the system 100 of FIG. 1 is illustrated. The OECT sensor 112 includes an organic electrochemical transistor. The OECT sensor 112 can include a channel material 222 that makes an electrical connection between a drain electrode 214 and a source electrode 216. For example, the channel material 222 between the drain electrode 214 and the source electrode 216 can be a conductive material. The channel material 222 can include a conjugated polymer. The channel material 222 can include a conductive polymer such as poly(3,4-ethylenedioxythiophene) polystyrene sulfonate (PEDOT:PSS).

The channel material 222 can be coated with a functionalized coating configured to facilitate detection of a small molecule drug. For example, the functionalized coating on the channel material 222 can change the conductivity of the channel material 222 in the presence of the drug of interest. For detecting a biomarker such as small molecule drug, the functionalized coating can include an aptamer or an antibody that selectively binds to the drug of interest. For example, binding of the drug molecules to either an aptamer coating or an antibody coating of the channel material 222 can alter the charge density at the interface between transistor channel 222 and analyte, which can change the conductivity of the channel material 222. In some other implementations, the gate electrode 218 of the OECT 112 may be functionalized with either an aptamer or an antibody, instead of the channel material 222. The change in gate electrode potential due to binding to target analytes will induce a change in conductivity through the channel material 222 can be detected in real-time, via the electrical contact pads 250a and 250b coupled respectively to the drain electrode 214 and the source electrode 216, or the electrical contact pads 256a and 256b coupled to the gate 218. For example, a current meter or voltage meter can probe the current or voltage via these contact pads. The magnitude of the current change indicates the amount of the drug present in a test sample. For example, a relatively large change in the current through the OECT sensor 112 can indicate a relatively large amount of the drug in the test sample. The OECT sensor 112, via the organic electrochemical transistor, can locally amplify an input signal before output and detection by the current meter.

In some implementations, the channel material 222 can have dimensions of about 2.5 mm by about 5.5 mm. The channel material 222 can overlap with each of the drain electrode 214 and the source electrode 215 by a lateral distance of about 100 µm. In some implementations, the channel material 222 may be significantly smaller. For example, the channel material 222 may have a length between 200 microns and 350 microns and a width between 2 microns and 6 microns. In some implementations, the channel material 222 may have a surface area that is smaller than a surface area of the gate electrode 218. For example, the gate electrode may have a surface area of that is two times, three times, four times, five times, six times, seven times, eight times, nine times, or ten times larger than the surface area of the channel material 222. In some implementations, the gate electrode may have a surface area that is 15 times, 20 times, 30 times, 50 times, or 100 times larger than the surface area of the channel material 222. In some implementations, the gate electrode may have a surface area that is 200 times, 300 times, 400 times, 500 times, 600 times, 700 times, 800 times, 900 times, or 1000 times larger than the surface area of the channel material 222.

In some implementations, the gate electrode 218 can be circular. The gate electrode 218 can have a diameter of about 1 mm, about 2 mm, about 3 millimeters. In some implementations, the gate electrode 218 can have a diameter between 1 mm and about 3 millimeters. In some implementations, the gate electrode 218 can have its center positioned about 2.0 mm or about 2.25 mm from the bottom of the channel material 222. In some implementations, the gate electrode 218 can be rectangular. In some implementations, the gate electrode 218 can have a length between 1 millimeter and 10 millimeters and a width between 3 millimeters and 7 millimeters. For example, in some implementations the gate electrode 218 can have dimensions of about 10 millimeters by about 5 millimeters, about 6 millimeters by about 5 millimeters, about 4 millimeters by about 5 millimeters, about 2 millimeters by about 5 millimeters, or about 1 millimeter by about 5 millimeters.

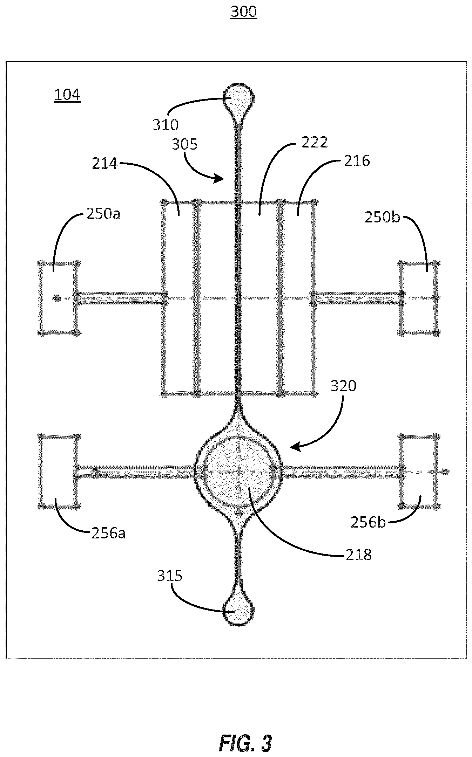

FIG. 3 illustrates a top view of an example system 300 for detecting drug levels in a fluid sample. The system 300 includes many of the components of the system 100 and the OECT sensor 112 depicted in FIGS. 1 and 2, and like reference numerals refer to like elements in these figures. The system 300 illustrated in FIG. 3 can include a microfluidic flow channel 305 configured to transport fluid, such as a blood sample or other fluid sample. A fluid sample can flow from an inlet 310 toward an outlet 315. Between the inlet 310 and the outlet 315, the fluid sample passes over components of the OECT sensor 112. The fluid sample can include any fluid that is to be tested for the drug. For example, the fluid sample can include blood, urine, saliva, sweat, or other bodily fluids as well. The fluid can contain the drug of interest, as well as toxins, bacteria, viruses, cells, particles, or any combination thereof. For example, blood can be extracted from a patient who has been prescribed the drug of interest, and may include formed elements such as erythrocytes (e.g., red blood cells), leukocytes (e.g., white blood cells), thrombocytes (e.g., platelets); bacteria; viruses; toxins; or any combination thereof.

The microfluidic channel 305 can include a region 320 that is substantially wider than a remainder of the microfluidic channel 305. In some implementations, the microfluidic channel 305 can have a width in the range of about 250 µm along a majority of its length. In some other implementations, the microfluidic channel 305 can have a width of about 500 µm along a majority of its length. In contrast, the region 320 may have a width equal to or larger than the width of the gate electrode 218, which may in the range of about 1 mm to about 2 mm.

As shown, the region 320 can be aligned with the gate electrode 218 of the OECT sensor 112. Thus, fluid flowing into the enlarged region 320 can have more surface area over which to contact the gate electrode 218, due to the larger width of the region 320 relative to the rest of the microfluidic channel 305. In this example, the gate electrode 218 can include a functionalized coating configured to interact with a target drug in the fluid sample. The increased surface area of the microfluidic channel 305 in the region 320 aligned with the gate electrode 218 can allow for increased interaction between the target drug within the fluid sample and the functionalized coating, thereby increasing the sensitivity of the system 300 to detection of the target drug.

Unlike the system 100 shown in FIG. 1, the system 300 may not include any separation regions. Rather, the fluid sample can be introduced at the inlet 310 and can flow to the outlet 315 without any components of the fluid sample being separated out. In some implementations, the inlet 310 and the outlet 315 can be configured for access by syringe tips that can be used to introduce or extract the fluid sample from the microfluidic channel 305.

Referring again to FIG. 1, the system 100 can include one or more separation regions 106 that can remove undesirable particles or cells from the sample fluid. For example, for a blood sample, the first separation region 106a can be configured to remove blood cells from the blood sample, and the second separation region 106b can be configured to remove bacteria such that substantially only plasma and particles of the drug of interest flow interact with the OECT sensor 112. The separation regions 106 can remove undesirable particles that can interfere with the OECT sensor's ability to detect the drug of interest. For example, blood cells or bacteria can impair the ability of the OECT sensor 112 to detect the drug of interest. Removal of the blood cells and bacteria via the separation regions 105 can therefore improve performance of the OECT sensor 112 for detecting the drug of interest. The removal of substances such as the blood cells and bacteria from the blood sample can enable a lower limit of detection (LOD) for the OECT sensor 112.

To remove particles from fluid flowing through the microfluidic flow channel, the system 100 can be coupled with one or more acoustic wave generators. For example, the system 100 can be coupled with a platform that positions an acoustic wave generator below each of the separation regions 106. The acoustic wave generators can each impart a respective standing wave across the separation regions 106. Particles (e.g., blood cells and bacteria cells) within the fluid sample can be driven towards the nodes or anti-nodes of the standing acoustic wave based the sign of the particles' contrast factor with respect to the fluid sample. For example, formed elements can have a positive contrast factor and can be driven, by the standing acoustic wave, towards the nodes of the standing acoustic wave. Particles with a negative contrast factor can be driven towards the antinode of the standing acoustic wave. The width of the microfluidic flow channel prior to the separation regions 106 and the placement of the acoustic wave generators can be configured such that the standing acoustic wave forms a node or antinode near the central, longitudinal axis of the microfluidic flow channel.

The first separation region 106a can drive the formed elements towards the central, longitudinal axis of the microfluidic flow channel (or other position of the standing acoustic wave's node) such that the formed elements exit the system 100 through a separation outlet in or at the end of the first separation region 106a. The first separation region 106a can drive the other components of the fluid sample, for example, bacteria, plasma, and virus toward the walls of the microfluidic flow channel such the components pass to the second separation region 106b. In some implementations, particles other than the formed elements (e.g., the bacteria, plasma, and virus) can also be driven towards the central, longitudinal axis (and outlet of the first separation region 106a), but at a rate slower than the formed elements. For these particles, the rate of movement towards the central, longitudinal axis may not be great enough to enable the particles to be sufficiently close to the central, longitudinal axis to exit through the first separation outlet and the particles can pass to the second separation region 106b.

The second separation region 106b can drive the remaining undesirable particles (e.g., bacteria) toward the central, longitudinal axis of the microfluidic flow channel such that the remaining particles exit the microfluidic flow channel through a second separation outlet. The remaining components of the fluid sample (e.g., the plasma, virus, and biomarkers) can flow to the OECT sensor, which may be defined within a well of the substrate 104.

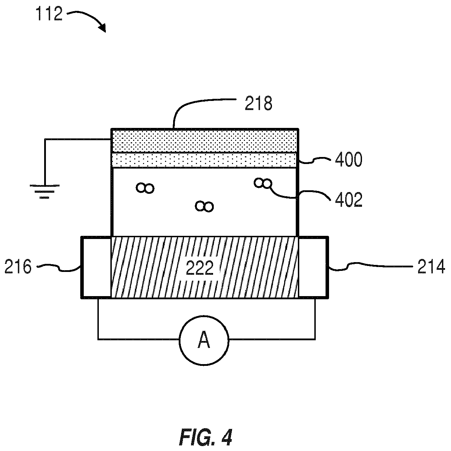

FIG. 4 illustrates a schematic diagram of an embodiment of the OECT sensor 112 that can be used in the system 100 of FIG. 1 or the system 300 of FIG. 3. In FIG. 4, the functionalized coating 400 (which can also be referred to as the coating 400) is applied to the gate electrode 218. The gate electrode 218 is illustrated as above the drain electrode 214 and the source electrode 216. The particles of the drug of interest 402 can be in a sample fluid that is in contact with the gate electrode 218, the source electrode 216, and the drain electrode 214. In some implementations, the gate electrode 218 can be in the substantially the same plane as the drain electrode 214 and the source electrode 216. For example, as illustrated in FIG. 2, the drain electrode 214, the source electrode 216, and the gate electrode 218 can each be components of a layer of material, which can be coupled to (or formed integrally with) the substrate 104 shown in FIG. 1.

The OECT sensor 112 includes the drain electrode 214, the source electrode 216, and the gate electrode 218. The drain electrode 214 and the source electrode 216 can be electrically coupled through the channel material 222. The drain electrode 214, the source electrode 216, the gate electrode 218, and the electrical traces 222 can include an electrically conductive metal such as gold, platinum, silver, or copper.

The channel material 222 can be a conductive polymer. The conductive polymer can include PEDOT:PSS. The channel material 222 can come into contact with both the drain electrode 214 and the source electrode 216 to form an electrochemical transistor. The channel material 222 can have a transconductance between about 2000 µs and about 5000 µs. The relatively high transconductance of the OECT sensor 112 can enable local amplification of an input signal before output and detection by a current or voltage meter.

In some implementations, the channel material 222 can be patterned to fill a void between the drain electrode 214 and the source electrode 216. For example, the drain electrode 214 and the source electrode 216 can first be patterned onto a substrate. Using a mask, the channel material 222 can be patterned into the space between the drain electrode 214 and the source electrode 216. The channel material 222 can be patterned to cover at least a portion of the drain electrode 214 and the source electrode 216. In contact with at least a portion of the drain electrode 214 and the source electrode 216, the channel material 222 can form an electrical connection between the drain electrode 214 and the source electrode 216.

The OECT sensor 112 can include a coating 400 that covers at least a portion of the gate electrode 218. The coating 400 can be a functionalized coating with a drug recognition element that interacts or bind to a drug to be detected in a fluid sample (i.e., the drug 402 shown in FIG. 4). An interaction of the drug 402 with drug recognition element can induce a change in the work function of the gate electrode 218, which can change the effective gate voltage. In some other implementations, the coating 400 may instead be applied to the channel material 222, such that interaction between the drug 402 and the coating 400 changes the conductivity of the channel material 222.

The coating 400 can change the conductivity of the channel material 222 or can change the work function of the gate electrode 218 (depending on the placement of the coating 400) in the presence of the drug 402. The coating 400 can include aptamers or antibodies. The drug 402 can transfer electrons to the coating 400 (or vice versa), which can induce a change in the work function of the gate electrode 218 (or change the conductivity of the channel material 222, if the coating 400 is instead applied to the channel material 222). The change can be detected in real-time by a current meter, for example.

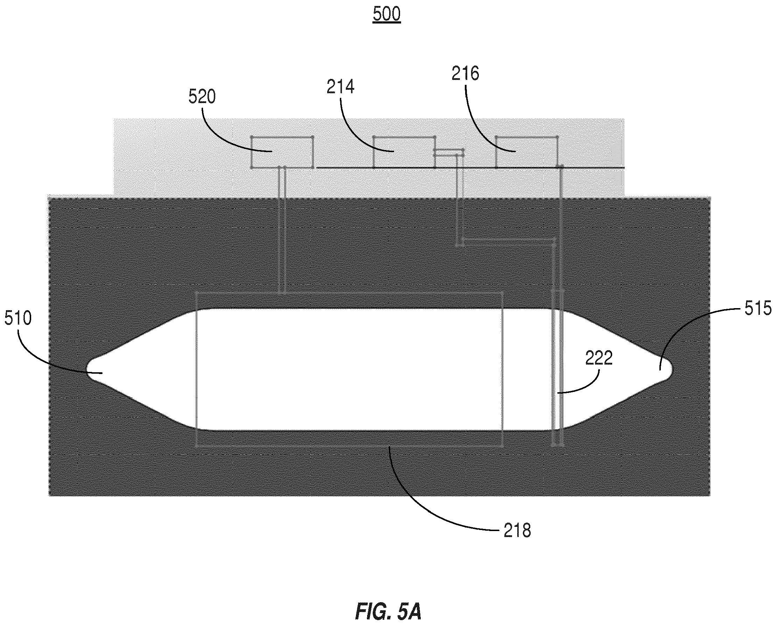

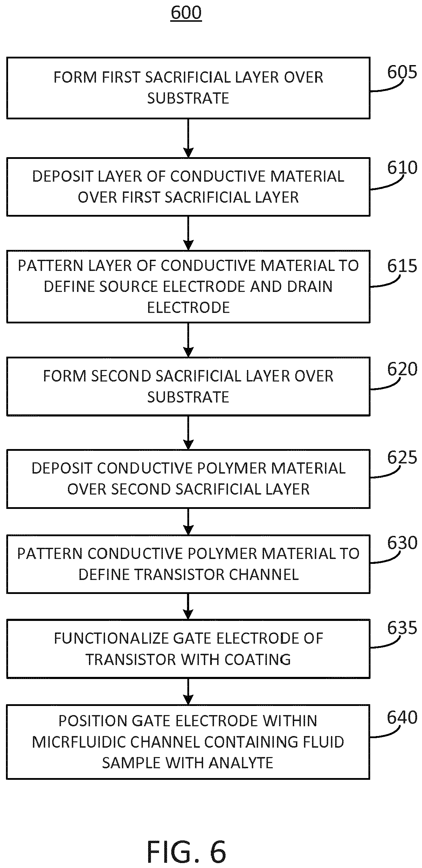

In some implementations, a device may be similar to the device 112 of FIGS. 2 and 3, but may have a gate electrode 218 that is formed separately from the source electrode 216, the drain electrode 214, and the channel 222. For example, the source electrode 216, the drain electrode 214, and the channel 222 can each be fabricated on a surface of a substrate such as the substrate 104. The gate electrode 218 can be electrically coupled with the other components of the transistor, but may not be fabricated on the substrate 104. As a result, the gate electrode 218 can be positioned outside of the plane defined by the surface of the substrate 104. Stated differently, the gate electrode 218 can be non-coplanar with the other components of the transistor, including the source electrode 216, the drain electrode 214, and the channel 222. This arrangement can allow the gate electrode to be positioned directly within the channel or reservoir that contains the fluid sample. Examples of such devices are described below in connection with FIGS. 5A-5C. An example technique for fabricating a device in this arrangement is described below in connection with FIG. 6.

FIG. 5A illustrates a top view of an embodiment of the organic electrochemical transistor sensor 500 that can be used in the system illustrated in FIG. 1. The OECT sensor 500 can include components of an OECT overlaid with microfluidic components. For example, a transistor can include a channel 222, a drain electrode 214, a source electrode 216, and a gate electrode 218. A microfluidic channel can extend along a flow channel between an inlet 510 and an outlet 515. As shown, the gate electrode 218 can be positioned within the flow channel. The transistor channel 222 can be positioned downstream from the gate electrode 218, between the gate electrode 218 and the outlet 515.

The transistor also can include a gate contact 520. In some implementations, the gate contact 520 can be formed on the same substrate as the channel 222, the drain electrode 214, and the source electrode 216. The gate contact 520 can also be electrically coupled with the gate electrode 218. In some implementations, separating the gate contact 520 from the gate electrode 218 can allow the gate electrode 218 to be formed in a separate process from the other components of the transistor. As a result, the gate electrode 218 can be more easily cleaned and functionalized with a coating that can include an aptamer or an antibody without impacting the other components of the transistor. In addition, the gate electrode 218 can be formed to be substantially larger than the gate contact 520. In some implementations, increasing the size of the gate electrode 518 relative to the size of the channel 222 can allow the OECT sensor 500 to have greater sensitivity to the analyte.

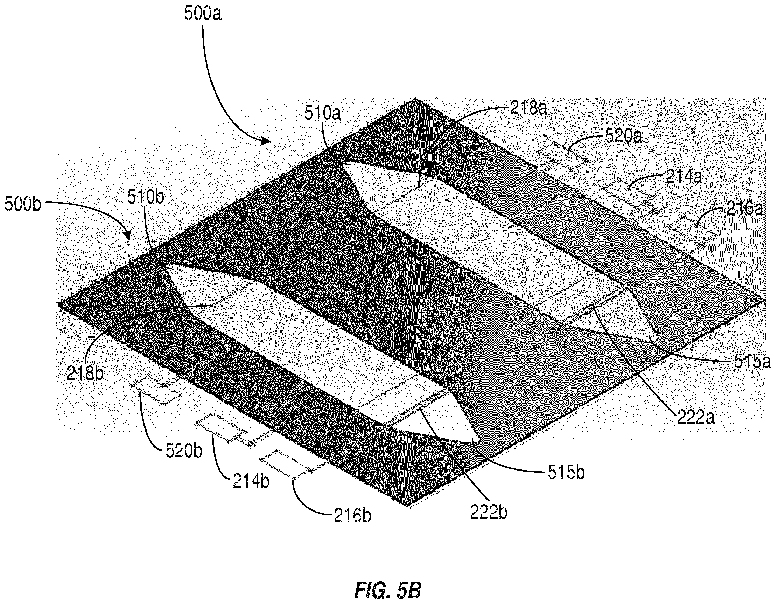

FIG. 5B illustrates a perspective view of two of the organic electrochemical transistor sensors of FIG. 5A arranged in parallel. As shown, the OECT sensor 500a is positioned adjacent to the OECT sensor 500b. Each of the OECT sensors 500a and 500b include components similar to those described above in connection with FIG. 5A, and like reference numerals refer to like elements in these figures. In some implementations, the OECT sensors 500a and 500b can be arranged such that the transistor components extend in opposite directions, thereby allowing the OECT sensors 500a and 500b to be positioned more closely together. In some implementations, the OECT sensors 500a and 500b can be manufactured substantially simultaneously on the same substrate or wafer. For example, a set of steps (e.g., photolithographic steps) for forming the components of the transistors of the OECT sensors 500a and 500b can be performed to fabricate the transistors for both the OECT sensor 500a and the OECT sensor 500b at the same time. In some implementations, additional OECT sensors may also be fabricated simultaneously. For example, although only two OECT sensors 500a and 500b are depicted in FIG. 5B, in some implementations any number of OECT sensors may be arranged in a grid on a common substrate or wafer and fabricated simultaneously.

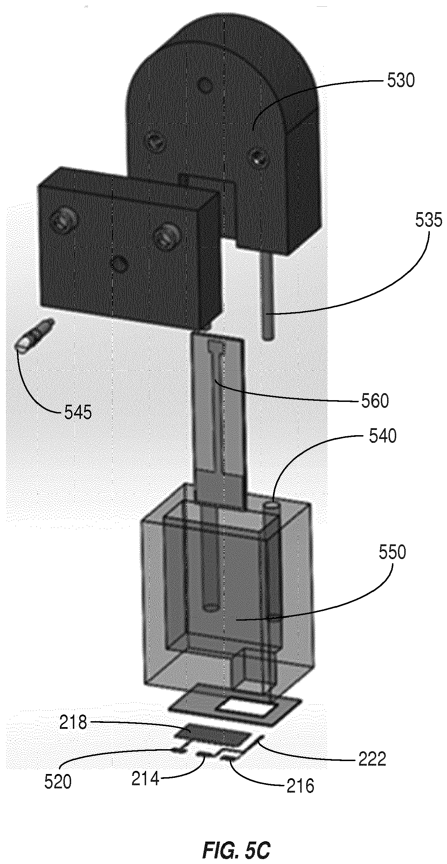

FIG. 5C illustrates an exploded view of an electrode fixture 530 that can be used in connection with the electrochemical transistor sensor 500 of FIG. 5A. The electrode fixture 530 can be configured to receive and secure an extended gate electrode 560. For example, the electrode fixture 530 includes two pieces that can be secured (e.g., via mechanical fasteners such as screws) to one another to clamp the extended gate electrode 560 in place. In some implementations, either or both of the pieces of the electrode fixture 530 can be formed using an additive manufacturing technique (e.g., 3D printing). In some implementations, the electrode fixture 530 can include an alignment pin 535, which can be inserted into a hole 540 formed in a substrate that includes a reservoir 550 to ensure proper alignment between the extended gate electrode 560 and the reservoir 550.

The fluid sample can be introduced into the reservoir 550 and can contact the extended gate electrode 560. As described above, an interaction between an analyte in the fluid sample and a functionalized coating on the gate electrode can alter a response of the transistor, which can be detected to determine a presence or concentration of the analyte in the fluid sample. A solderless electrical contact 545 can be inserted through a portion of the electrode fixture 530 to contact the extended gate electrode 560. Thus, electrical equipment can be coupled with the extended gate electrode 560 via the solderless contact pin 545 to eliminate the need to solder the extended gate electrode 560. This can allow for cleaning and functionalization of the extended gate electrode 560 to be performed as a more controlled process, and can help to reduce error in drain current response.

In some implementations, the extended gate electrode 560 can also allow the distance between the gate electrode and the contact pad (e.g., the distance between the extended gate electrode 560 and the solderless contact pin 545) to be increased, relative to the distance between the gate electrode 218 and the contact pad 520 formed on the substrate with the other components of the transistor. For example, in some implementations the distance between the gate electrode 218 and the contact pad 520 can be about 5 millimeters, while the distance between the extended gate electrode 560 and the solderless contact pin 545 can be about 20 millimeters.

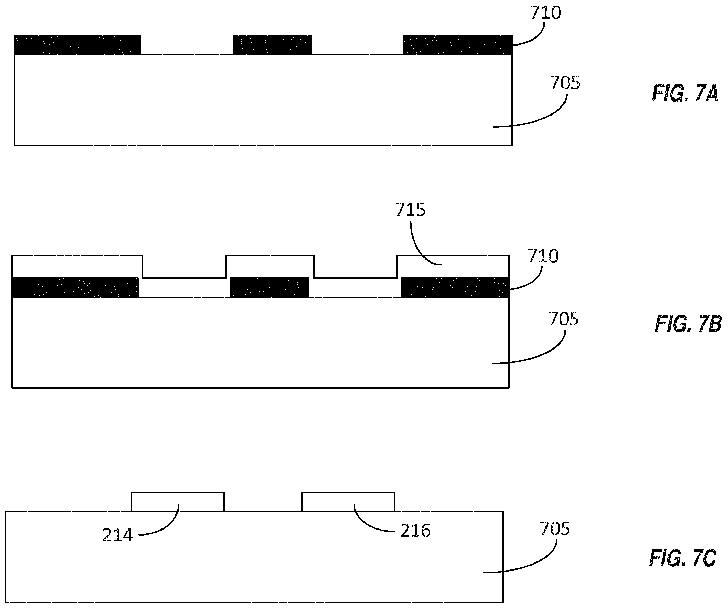

FIG. 6 illustrates a flowchart of an embodiment a method 600 for fabricating a device for detecting an analyte in a fluid sample. FIGS. 7A-7F illustrate stages of construction of a portion of an example device that can be fabricated according to the method of FIG. 6. FIGS. 6 and 7A-7F are therefore described together below. Referring now to FIG. 6, the method 600 can include forming a first sacrificial layer over a substrate (BLOCK 605). The result of this stage is shown in FIG. 7A, in which the first sacrificial layer 710 has been formed on the substrate 705. In some implementations, the substrate 705 can be or can include at least one of glass, Pyrex, acrylic, or polydimethylsiloxane (PDMS). The first sacrificial layer 710 can be formed from one or more layers of material deposited over the surface of the substrate 705 and patterned according to a desired shape of the first sacrificial layer 710. In some implementations, the first sacrificial layer 710 can be formed using photolithographic techniques. For example, the first sacrificial layer 710 can include one or more layers of photoresist. In some implementations, the one or more layers of photoresist can be deposited on the substrate using a technique such as spin coating the substrate with the photoresist. In some implementations, the photoresist can be cured. The one or more layers of photoresist can then be shaped or patterned to form a mold (or a negative mold) for portions of the device to be fabricated later. For example, the first sacrificial layer 710 can be patterned using photolithographic techniques to have a shape defining openings that correspond to components of a transistor, which may include a drain electrode and a source electrode.

The method 600 can include depositing a layer of conductive material over the first sacrificial layer 710 (BLOCK 610). The results of this stage are shown in FIG. 7B. As illustrated, the conductive material 715 can be deposited conformally over the first sacrificial layer 710. In some implementations, the layer of conductive material 715 can be spin coated over the first sacrificial layer 710. Because the first sacrificial layer 710 includes openings that expose the underlying substrate 705, a portion of the conductive material 715 can be in contact with the substrate 705. In some implementations, the conductive material 715 can include chromium, gold, copper, platinum, or any other conductive metal or alloy. In some implementations, the conductive material 715 can be selected to adhere to the substrate 705.

In some implementations, the conductive material 715 can include more than one layer of material. For example, a first layer of conductive material, such as chromium, may be deposited first. Then a second layer of another conductive material, such as gold, may be deposited over the first layer of conductive material. In some implementations, the first layer of conductive material may be deposited to a thickness of about 100 nm, while the second layer of conductive material may be deposited to a thickness of about 300 nm.

The method 600 can include patterning the layer of conductive material to define the source electrode and the drain electrode of the transistor (BLOCK 615). The results of this stage are shown in FIG. 7C. As depicted, the portions of the conductive material 715 that were in contact with the substrate 705 after the conductive material 715 was deposited over the first sacrificial layer 710 can remain on the substrate 705, while other portions of the conductive material 715 are removed during the patterning process. The remaining portions of the conductive material 715 can form the source electrode 216 and the drain electrode 214. In some implementations, the sacrificial layer 710 itself also can be removed. Either or both of the sacrificial layer 710 and the undesired portions of the conductive material 715 can be removed using lithographic techniques, including photolithographic patterning and lift-off. In some implementations, a lift-off process can be used to form the pattern in the layer of conductive material 715. For example, lift-off of the underlying sacrificial layer 710 can cause the undesired portions of the layer of conductive material 715 (e.g., portions that do not correspond to the source electrode 216, the drain electrode 214, or other components of the transistor) to be removed as well, thereby resulting in patterning of the layer conductive material 715 to form the components of the transistor.

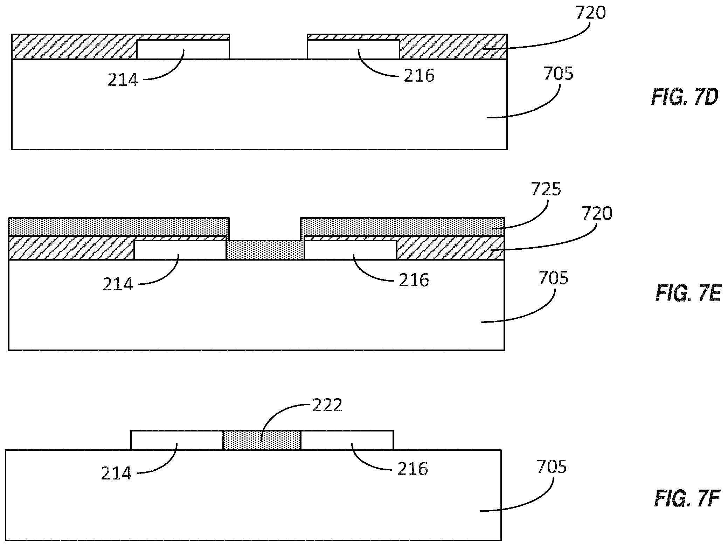

The method 600 can include forming a second sacrificial layer over the substrate (BLOCK 620). The result of this stage is shown in FIG. 7D, in which the second sacrificial layer 720 has been formed over the substrate 705 as well as the drain electrode 214 and the source electrode 216. In some implementations, second sacrificial layer 720 can be formed from one or more layers of material deposited and patterned according to a desired shape of the second sacrificial layer 720. In some implementations, the second sacrificial layer 720 can be formed using photolithographic techniques. For example, the second sacrificial layer 720 can include one or more layers of photoresist. In some implementations, the one or more layers of photoresist can be deposited using a technique such as spin coating of the photoresist. In some implementations, the photoresist can be cured. The one or more layers of photoresist can then be shaped or patterned to form a mold (or a negative mold) for portions of the device to be fabricated later. For example, the second sacrificial layer 720 can be patterned using photolithographic techniques to have a shape defining at least one opening that corresponds to a channel of the transistor. For example, the second sacrificial layer 720 can include an opening positioned between the source electrode 216 and the drain electrode 214, as shown. The second sacrificial layer 720 can be formed from a material, such as a photoresist, that is selected to adhere to any of the substrate 705, the source electrode 216, or the drain electrode 214.

The method 600 can include depositing a conductive polymer material over the second sacrificial layer (BLOCK 625). The results of this stage are shown in FIG. 7E. As illustrated, the conductive polymer 725 can be deposited conformally over the second sacrificial layer 720. In some implementations, the conductive polymer 725 can be spin coated over the second sacrificial layer 720. Because the second sacrificial layer 720 includes openings that expose the underlying substrate 705, a portion of the conductive polymer 725 can be in contact with the substrate 705. In some implementations, the conductive polymer 725 can include PEDOT:PSS. In some implementations, the conductive polymer 725 can be selected to adhere to the substrate 705.

The method 600 can include patterning the conductive polymer material to define the transistor channel (BLOCK 630). The results of this stage are shown in FIG. 7F. As depicted, the portions of the conductive polymer 725 that were in contact with the substrate 705 after the conductive polymer 725 was deposited over the second sacrificial layer 720 can remain on the substrate 705, while other portions of the conductive polymer 725 are removed during the patterning process. The remaining portions of the conductive polymer 725 can form the channel 222. In some implementations, the second sacrificial layer 720 itself also can be removed. Either or both of the second sacrificial layer 720 and the undesired portions of the conductive polymer 725 can be removed using lithographic techniques, including photolithographic patterning and lift-off. In some implementations, a lift-off process can be used to form the pattern in the conductive polymer 725. For example, lift-off of the underlying second sacrificial layer 720 can cause the undesired portions of the conductive polymer 725 (e.g., portions that do not correspond to the channel 222 of the transistor) to be removed as well, thereby resulting in patterning of the conductive polymer 725 to form the channel 222 of the transistor.

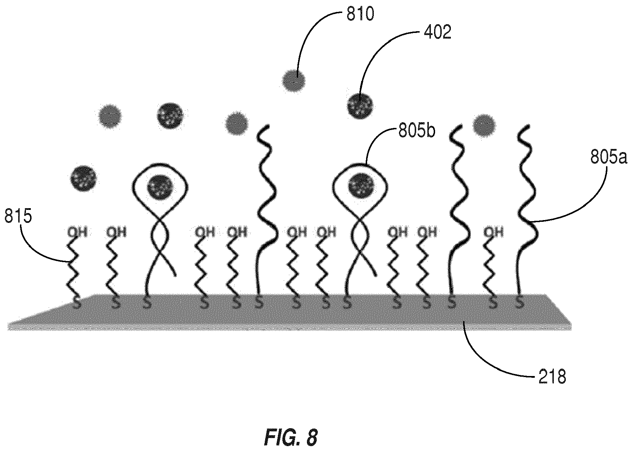

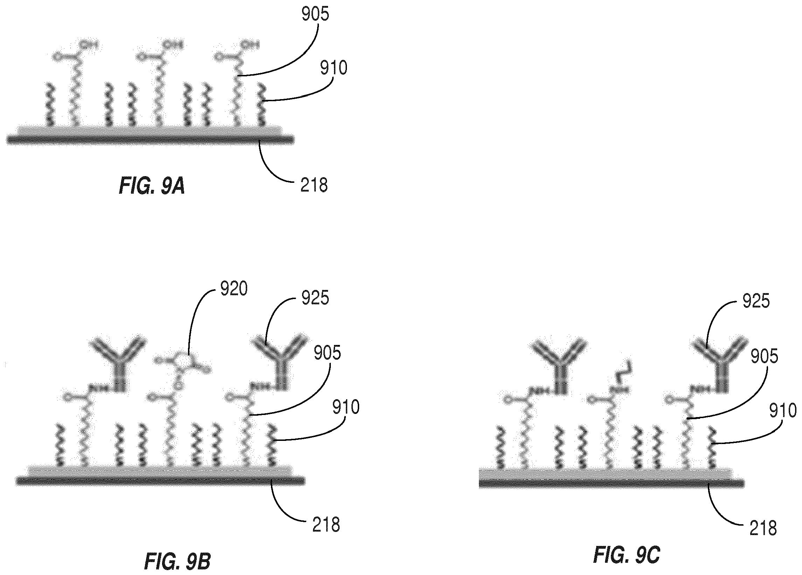

The method 600 can include functionalizing a gate electrode of the transistor with transistor coating (BLOCK 635). In some implementations, the gate electrode can be formed separately from the other components of the transistor, such as the source electrode 216, the drain electrode 214, and the channel 222. For example, the gate electrode may not be formed on the substrate 705. Instead, the gate electrode can be formed separately and later can be electrically coupled with other components of the transistor on the substrate 705. In some implementations, the gate electrode can be functionalized with a coating such as an aptamer or an antibody, which may selected to bind with the analyte (e.g., a target drug molecule) to change a work function of the gate electrode. In some implementations, the gate electrode can be an extended gate electrode such as the extended gate electrode 560 shown in FIG. 5C. Techniques for applying such a functional coating to a gate electrode are described further below in connection with FIGS. 8 and 9A-9C.

The method 600 can include positioning the gate electrode within a microfluidic channel containing the fluid sample with the analyte (BLOCK 640). In some implementations, the microfluidic channel may be formed on or defined by the substrate 705. In some other implementations, the microfluidic channel may be formed separately from the substrate 705. For example, the microfluidic channel can be formed from a PDMS, acrylic, or glass material, which may then be bonded or otherwise integrated with the substrate 705. When a fluid sample is introduced into the channel, an output of the transistor may be altered in a manner that corresponds to a presence or level (e.g., concentration) of the analyte of interest within the fluid sample.

FIG. 8 illustrates a cross-sectional view of a gate electrode of an example OECT sensor functionalized with an aptamer, which can be used in the system 100 illustrated in FIG. 1 or the system 300 illustrated in FIG. 3. In this example, the gate electrode 218 is functionalized with an aptamer 805 selected to bind with the target drug 402. The gate electrode 218 can include a surface formed from gold. To functionalize the gold surface with the aptamers 805, the gold surface may be cleaned, for example using an oxygen plasma cleaning or electrochemical cleaning technique. For example, a cyclic voltammetry scan can be performed in 0.5 M H₂SO₄. The aptamers 805 can be folded and prepared for surface immobilization to generate functional thiols.

In some implementations, the aptamers 805 can be RNA or DNA aptamers. For example, the aptamers 805 can be single stranded DNAs created as specific ligands for the drug of interest. The thiol-functionalized aptamers 805 can be mixed with 6-mercapto-hexanol (MCH) in a phosphate buffer. For example, the ratio aptamers to MCH can be about 1:100 in the buffer. The gold surface can then be immersed in or otherwise exposed to this solution. The gold surface can then be backfilled with a high concentration of MCH and washed and stored in a phosphate buffer.

Thus, the functionalized coating (i.e., the coating 400 shown in FIG. 4) can include aptamers 805 and MCH 815 shown in FIG. 8, positioned on the surface of the gate electrode 218. When the sample fluid (e.g., a blood sample) contacts the functionalized gate electrode 218, the drug 402 can bind with the aptamers 805. For example, the aptamer 805b is shown bound to a particle of the drug 402, while the aptamer 805a is shown in an unbound state. The fluid sample may also include other non-target particles 810, which do not bind with the aptamers 805. As the fluid flows over the gate electrode 218 and the aptamers 805 bind with particles of the target drug 402, the output of the OECT can change accordingly, as described above. Thus, the presence of the drug 402 can be detected. The magnitude of the change in the output of the OECT can indicate a level (e.g., a concentration) of the target drug 402 in the sample.

As described above, drug detection can also be achieve through the use of a functionalized coating that includes an antibody selected to interact with the drug of interest. FIGS. 9A-9C illustrates cross-sectional views of stages of functionalization of a gate electrode 218 of an example OECT sensor functionalized with an antibody, which can be used in the system 100 illustrated in FIG. 1 or the system 300 illustrated in FIG. 3. In some implementations, the gate electrode 218 can include a surface formed from gold, or another conductive metal. The gold surface can be cleaned in a manner similar to that described above in connection with FIG. 8, for example via oxygen plasma cleaning or electrochemical cleaning.

The gold surface can then be functionalized with a mixture of active groups 905 (which may include alkyl thiols terminated with carboxylic acids), as well as inactive spacer groups 910 (which may include MCH), as shown in FIG. 9A. The carboxylic acids can be activated with an activation agent 920, which may be a carbodiimide such as EDCI and/or N-hydroxysuccinimide (NHS), as shown in FIG. 9B. Antibodies 925 can then be added, forming covalent bonds between amines on antibody lysine residues and the activated acids 905 on the surface of the gate electrode 218. Then, the surface can be washed to remove the unbound activation agent 920, and stored in a phosphate buffer. The results of this stage are shown in FIG. 9C.

Thus, the functionalized coating (i.e., the coating 400 shown in FIG. 4) can include antibodies 925 as shown in FIG. 9C, positioned on the surface of the gate electrode 218. When the sample fluid (e.g., a blood sample) contacts the functionalized gate electrode 218, the drug 402 can bind with the antibodies 925. As the fluid flows over the gate electrode 218 and the antibodies 925 bind with particles of the target drug 402, the output of the OECT can change accordingly, as described above. Thus, the presence of the drug 402 can be detected. The magnitude of the change in the output of the OECT can indicate a level (e.g., a concentration) of the target drug 402 in the sample.

While operations are depicted in the drawings in a particular order, such operations are not required to be performed in the particular order shown or in sequential order, and all illustrated operations are not required to be performed. Actions described herein can be performed in a different order.

The separation of various system components does not require separation in all implementations, and the described program components can be included in a single hardware or software product.

Having now described some illustrative implementations, it is apparent that the foregoing is illustrative and not limiting, having been presented by way of example. In particular, although many of the examples presented herein involve specific combinations of method acts or system elements, those acts and those elements may be combined in other ways to accomplish the same objectives. Acts, elements and features discussed in connection with one implementation are not intended to be excluded from a similar role in other implementations or implementations.

The phraseology and terminology used herein is for the purpose of description and should not be regarded as limiting. The use of "including" "comprising" "having" "containing" "involving" "characterized by" "characterized in that" and variations thereof herein, is meant to encompass the items listed thereafter, equivalents thereof, and additional items, as well as alternate implementations consisting of the items listed thereafter exclusively. In one implementation, the systems and methods described herein consist of one, each combination of more than one, or all of the described elements, acts, or components.

As used herein, the term "about" and "substantially" will be understood by persons of ordinary skill in the art and will vary to some extent depending upon the context in which it is used. If there are uses of the term which are not clear to persons of ordinary skill in the art given the context in which it is used, "about" will mean up to plus or minus 10% of the particular term.

Any references to implementations or elements or acts of the systems and methods herein referred to in the singular may also embrace implementations including a plurality of these elements, and any references in plural to any implementation or element or act herein may also embrace implementations including only a single element. References in the singular or plural form are not intended to limit the presently disclosed systems or methods, their components, acts, or elements to single or plural configurations. References to any act or element being based on any information, act or element may include implementations where the act or element is based at least in part on any information, act, or element.

Any implementation disclosed herein may be combined with any other implementation or embodiment, and references to "an implementation," "some implementations," "one implementation" or the like are not necessarily mutually exclusive and are intended to indicate that a particular feature, structure, or characteristic described in connection with the implementation may be included in at least one implementation or embodiment. Such terms as used herein are not necessarily all referring to the same implementation. Any implementation may be combined with any other implementation, inclusively or exclusively, in any manner consistent with the aspects and implementations disclosed herein.

The indefinite articles "a" and "an," as used herein in the specification and in the claims, unless clearly indicated to the contrary, should be understood to mean "at least one."

References to "or" may be construed as inclusive so that any terms described using "or" may indicate any of a single, more than one, and all of the described terms. For example, a reference to "at least one of 'A' and 'B'" can include only 'A', only 'B', as well as both 'A' and 'B'. Such references used in conjunction with "comprising" or other open terminology can include additional items.

Where technical features in the drawings, detailed description or any claim are followed by reference signs, the reference signs have been included to increase the intelligibility of the drawings, detailed description, and claims. Accordingly, neither the reference signs nor their absence has any limiting effect on the scope of any claim elements.

The systems and methods described herein may be embodied in other specific forms without departing from the characteristics thereof. The foregoing implementations are illustrative rather than limiting of the described systems and methods. Scope of the systems and methods described herein is thus indicated by the appended claims, rather than the foregoing description, and changes that come within the meaning and range of equivalency of the claims are embraced therein.

## Claims

1. A microfluidic device for detecting an analyte in a fluid sample, the microfluidic device comprising:
a substrate defining a flow channel configured to transport the fluid sample from an inlet of the flow channel to an outlet of the flow channel;
an organic electrochemical transistor (OECT) comprising a source, a drain, a transistor channel, and a gate, wherein at least one of the transistor channel or the gate of the OECT overlaps a portion of the flow channel to contact the fluid sample in the flow channel;
a coating applied to at least one of the transistor channel or the gate, the coating comprising an aptamer or an antibody selected to bind with the analyte to change a conductivity of the transistor channel or a work function of the gate; and
a sensor configured to receive an electrical output of the OECT and to detect a level of the analyte within the fluid sample based on the electrical output of the OECT.

2. The microfluidic device of claim 1, further comprising a first separation region positioned in the flow channel between the inlet and the OECT, the first separation region configured to remove cells from the fluid sample before the fluid sample flows to the OECT.

3. The microfluidic device of claim 2, wherein the first separation region comprises a separation outlet configured to receive a portion of the fluid sample containing the cells and to transport the portion of the fluid sample containing the cells away from the flow channel.

4. The microfluidic device of claim 1, wherein the transistor channel of the OECT comprises a conductive polymer material.

5. The microfluidic device of claim 1, wherein the gate of the OECT further comprises a gate electrode comprising a gold surface positioned within the flow channel of the microfluidic device.

6. The microfluidic device of claim 1, wherein:
a majority of a length of the flow channel has a first width; and
a region of the flow channel surrounding the gate of the OECT has a second width that is at least twice the first width.

7. The microfluidic device of claim 1, wherein the transistor channel of the OECT has a length between 200 microns and 350 microns and a width between 2 mm and 6 mm.

8. The microfluidic device of claim 1, wherein the gate of the OECT has a rectangular shape with a length between 1 millimeter and 10 millimeters and a width between 3 millimeters and 7 millimeters.

9. The microfluidic device of claim 1, wherein the analyte comprises a small molecule drug.

10. The microfluidic device of claim 1, wherein the small molecule drug comprises carbamazepine.

11. A method of fabricating a device for detecting an analyte in a fluid sample, the method comprising:
forming a first sacrificial layer on a surface of a substrate, the first sacrificial layer patterned for the deposition of a source electrode and a drain electrode of a transistor;
depositing a layer of conductive material over the first sacrificial layer;
patterning the layer of conductive material to define the source electrode and the drain electrode of the transistor;
forming a second sacrificial layer over the substrate, the second sacrificial layer patterned for the deposition of a transistor channel;
depositing a conductive polymer material over the second sacrificial layer;
patterning the conductive polymer material to define the transistor channel;
functionalizing a gate electrode of the transistor with a coating comprising an aptamer or an antibody selected to bind with the analyte to change a work function of the gate electrode; and
positioning the gate electrode within a microfluidic channel containing the fluid sample with the analyte.

12. The method of claim 11, further comprising:
introducing the fluid sample containing the analyte into an inlet of the microfluidic channel;
receiving an electrical output of the transistor; and
detecting a level of the analyte within the fluid sample based on the electrical output of the transistor.

13. The method of claim 11, wherein depositing the layer of conductive material over the first sacrificial layer comprises depositing a layer of gold.

14. The method of claim 11, wherein depositing the conductive polymer material over the second sacrificial layer comprises depositing a layer of poly(3,4-ethylenedioxythiophene) polystyrene sulfonate (PEDOT:PSS).

15. The method of claim 11, further comprising:
cleaning a surface of the gate electrode using at least one of oxygen plasma cleaning or electrochemical cleaning; and
applying the coating to the surface of the gate electrode after the surface of the gate electrode is cleaned.
